# EUROPEAN PATENT SPECIFICATION

(11) **EP 0 780 133 B1**
(45) Date of publication and mention of the grant of the patent: **08.03.2000**
(21) Application number: 95830538.5
(22) Date of filing: 22.12.1995
(51) Int. Cl.: A61M 1/16, A61M 1/28

(54) **Dialysis unit for purifying blood, particularly in patients affected with organ failure**
Dialysevorrichtung für Reinigung von Blut, speziell bei Patienten mit Organversagen
Appareil de dialyse pour purifier le sang, en particulier pour les patients atteints de déficience d'un organe

(43) Date of publication of application: 25.06.1997
(60) Divisional application: 99111638.5
(73) Proprietor: BELLCO S.p.A., 41037 Mirandola (IT)
(72) Inventor: Wratten Mary Lou, 41036 Medolla (IT); Tetta Ciro, 41037 Mirandola (IT)
(74) Representative: Plebani, Rinaldo

(56) References cited:
- EP-A- 0 278 465
- EP-A- 0 331 505
- EP-A- 0 355 847
- EP-A- 0 451 429
- EP-A- 0 461 333
- DE-A- 3 527 411

## Description

The present invention relates to a highly effective dialysis unit for purifying blood, particularly in patients affected with acute and/or chronic renal failure.

In patients suffering from organ failure, the blood gradually retains endless quantities of various substances commonly referred to as "toxins", and which, in healthy subjects, the relative organs are normally capable of eliminating from the bloodstream. The accumulation of such toxins eventually results in what is commonly known as organ failure, which, if not treated, results in various forms of damage and eventually in death. The most commonly used systems for combatting the syndrome consist in purifying the blood of the toxins by adsorbing them on solid media (hemo- and plasmaperfusion), or by ultrafiltering the blood or plasma through appropriate semipermeable membranes, either by convection with the aid of a pressure gradient (TMP) through the membrane (hemo- or plasmafiltration), or by diffusion by bringing the blood or plasma to be purified into contact with one side of the membrane, and an appropriately formulated wash solution into contact with the opposite side (hemodialysis).

All the above systems, however, present drawbacks. Hemoperfusion consists in percolating the blood directly through a filter of adsorbent material, which must therefore be made highly biocompatible. This is usually achieved by covering the adsorbent particles with appropriate material, which, however, seriously impairs the toxin-retaining capacity of the particles. In the case of plasmaperfusion, the blood is first filtered to separate the plasma, which is then percolated through the adsorbent material. Though this to some extent solves the problem of biocompatibility during perfusion, the increase in the viscosity of the blood during filtration may result in extensive clotting through the membrane, so that in any case the blood must be treated with anticoagulants (heparin).

Hemo- and plasmafiltration, on the other hand, only provide for removing high-molecular-weight toxins, and produce a considerable weight loss which must be compensated by feeding an infusion solution into the patient's blood. According to European Patent Application EP-A-0451429, the above problem may be partly solved by regenerating the ultrafiltrate, by absorbing the medium-high-molecular-weight toxins in it by percolating it through bare activated-carbon-based hemoperfusion cartridges such as DETOXIL 2™ produced by SORIN BIOMEDICA of Saluggia, Italy, so that the regenerated ultrafiltrate may be used, as it is or with additions, as an infusion solution.

Hemodialysis, particularly if combined with one or more of the above methods, is the most effective, but is relatively lengthy in terms of purification time, and therefore fails to provide for sufficient toxin removal in acute crises. Moreover, since certain hydrophobic substances such as the platelet activating factor (PAF), endotoxins, bilirubin, hippurates, protein-bound toxins such as indoxyl-sulfate, and free molecular oxygen radicals are only removed poorly if at all, eventual organ failure at present can be no more than delayed as opposed to fully prevented.

It is an object of the present invention to overcome the aforementioned drawbacks by providing an improved dialysis unit for removing any and all toxins, particularly both hydrosoluble and liposoluble, from blood.

According to the present invention, there is provided a dialysis unit for purifying blood, particularly in patients affected with organ failure as claimed in claim 1.

A number of non-limiting embodiments of the present invention will be described by way of example with reference to the accompanying drawings, in which:
Figure 1 shows, schematically, the operating principle of the dialysis unit according to the present invention;
Figures 2 and 3 show, schematically, two embodiments of an experimental dialysis circuit according to the present invention;
Figure 4 shows, schematically, the basic principle of an improved variation of the dialysis unit according to the present invention;
Figure 5 shows a schematic view of a dialysis unit in accordance with the present invention;
Figures 6 and 7 show test results of the Figure 2 and 3 circuits;
Figure 8 shows, schematically, the operating principle of the known dialysis method.

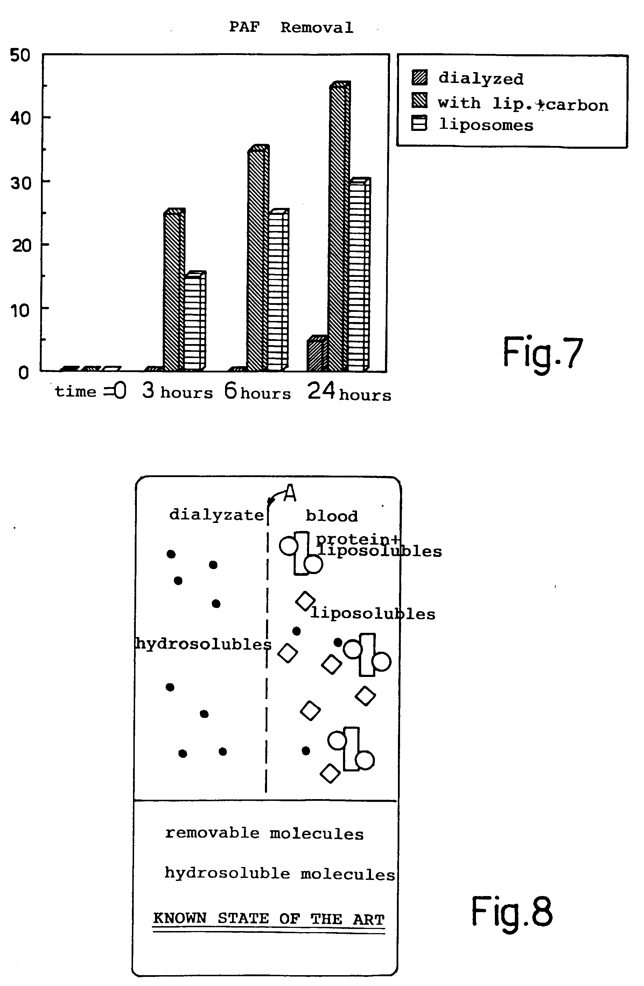

With reference to Figure 8, the known dialysis method, on which known units for treating patients affected with organ failure are based, consists simply in removing by diffusion through a semipermeable membrane A the hydrosoluble molecules (shown by the dark circles) contained (to the right of membrane A) in a stream of blood (or plasma), and transferring them into a stream of dialyzate (to the left of membrane A) comprising an appropriate aqueous solution. As such, however, any harmful liposoluble molecules to the right of membrane A, and any protein-bound molecules in the bloodstream are left in the patient's blood.

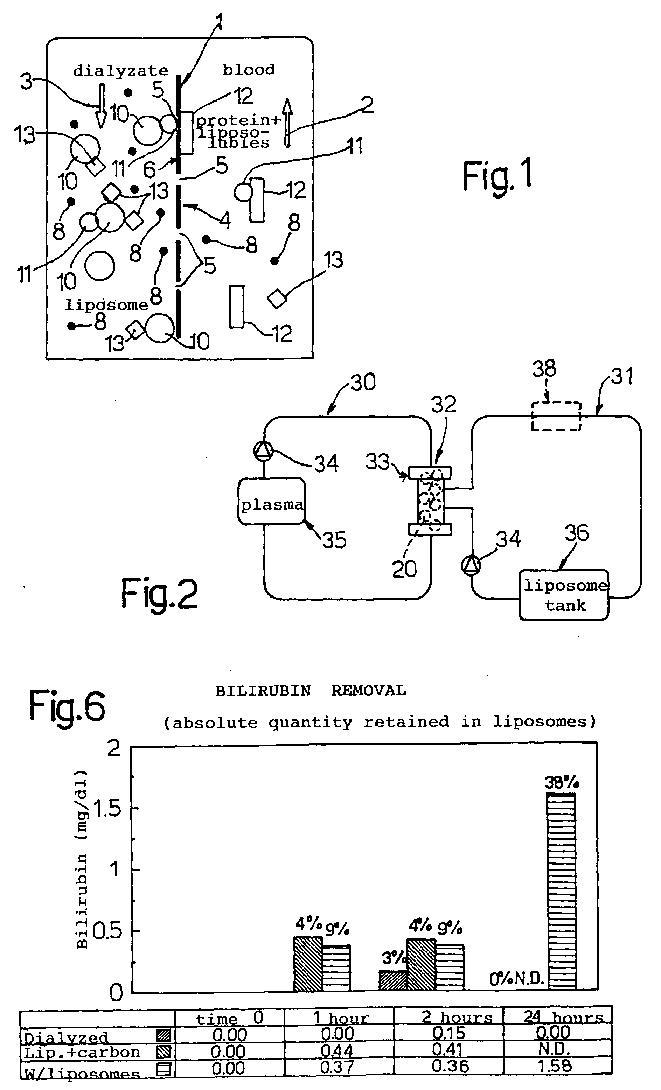

With reference to Figure 1, the operating principle of the dialysis unit according to the invention is as follows: a stream 2 (shown by the arrow) of a colloidal solution for purification (e.g. whole blood or an ultrafiltrate or plasmafiltrate of whole blood) is brought into contact with one side 4 of a semipermeable membrane 1 presenting a number of pores 5 of a given size and arrangement; and a stream 3 (shown by the arrow) of dialyzate, comprising a known aqueous wash solution of given composition, is brought into contact with the opposite side 6 of membrane 1, preferably flowing the opposite way to stream 2, so that the hydrosoluble target molecules 8 in colloidal solution 2, e.g. low-molecular-weight toxins, pass through the pores 5 of membrane 1 (which constitutes the interface between the two contacting phases) and diffuse from solution 2 into dialyzate 3.

According to the present invention, a predetermined concentration of liposomes 10, generally larger than the pores 5 of membrane 1, is fed into wash solution 3. According to the invention, therefore, dialysis is performed in the usual way, but using a dialyzate comprising a wash solution in turn comprising a water-base solvent (containing or not appropriate solutes) in which liposomes 10 are dispersed in suspension, preferably with a concentration, in correspondence with membrane 1, of 1 to 20 g/l. The liposomes 10 used according to the invention comprise rounded vesicles ranging from 100 nm to 200 nm in diameter, and defined by phospholipid membranes, e.g. with a lecithin base or equivalent (phosphatidylserine, dipalmitolphosphatidylcholine, etc.).

As shown schematically in Figure 1, liposomes 10 in dialyzate 3 provide for removing from solution 2 not only hydrosoluble molecules 8, but also a wide range of liposoluble target molecules (toxins) such as toxins 11 (e.g.indoxyl-sulfate) normally bound to respective proteins 12 in the bloodstream, low/medium-molecular-weight hemodialysis substances 13 (bilirubin, hippurates, PAF, probably endotoxins, overdoses of liposoluble drugs) and free radicals. In fact, in contact with membrane 1, liposomes 10 adjacent to pores 5 (through which they are too large to pass into stream 2) increase the hydrophobicity of membrane 1 on the stream 3 side.

Liposomes 10 therefore provide for safely capturing and incorporating wholly or partly in their lipid membranes any molecules 13 approaching pores 5, and for drawing them from stream 2 into dialyzate stream 3. Similarly, on account of the increased hydrophobicity of membrane 1, proteins 12 are brought into close contact with the membrane, thus enabling liposoluble molecules 11 bound to them to approach pores 5 where they are captured by liposomes 10 (top of Figure 1) and drawn out of stream 2. The same operating principle also applies to any free radicals in stream 2.

To remove the free radicals even more effectively from stream 2, or at least fully neutralize them, according to a preferred variation of the invention, it is also possible to incorporate in known manner into liposomes 10 one or more antioxidants, in particular vitamin E or ubiquinone/ubiquinol, either singly or mixed. In which case, even in the event the free radicals fail to be drawn into stream 3, they are nevertheless neutralized, on contacting liposomes 10 through pores 5 of membrane 1, by the antioxidant in the liposomes, e.g. in the phospholipid membrane, or inside the membrane itself, possibly in the form of an aqueous-phase solution trapped inside the vesicle defined by the phospholipid membrane.

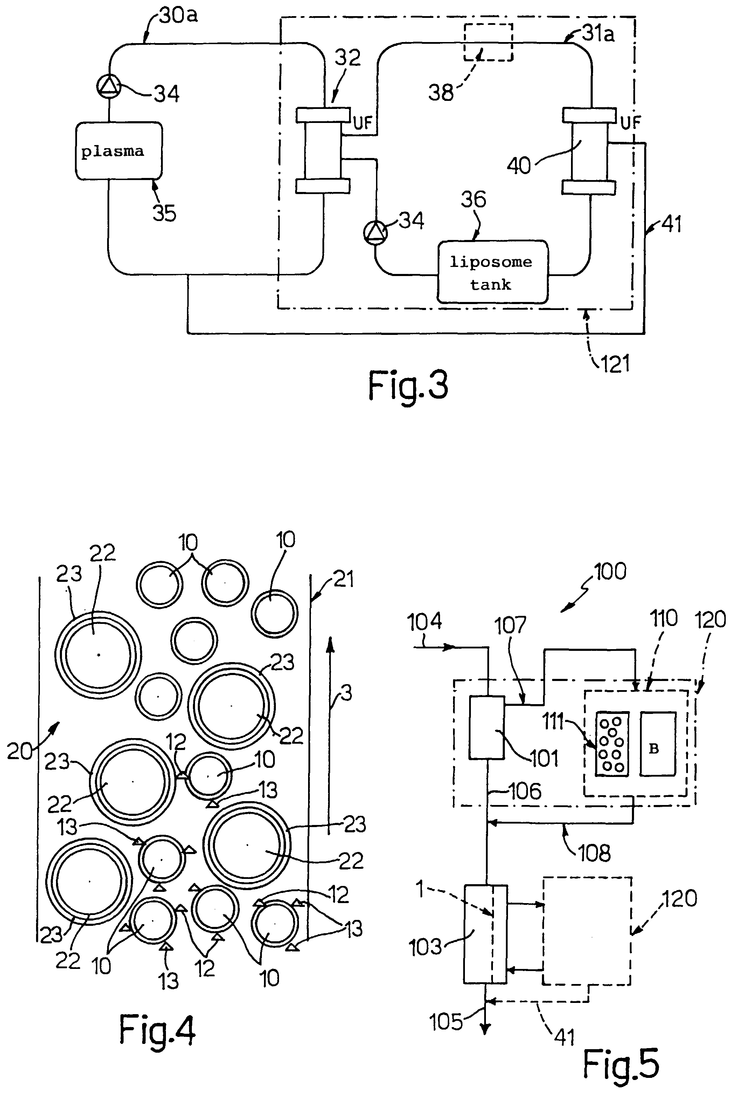

According to a further preferred variation of the invention, wash solution 3, fed onto membrane 1 in the opposite direction to the incoming stream comprising colloidal solution 2, may be subsequently percolated (Figure 4) through a filter 20 of highly permeable hollow fibers filled with adsorbent material on the extracapillary side, the filter possibly being housed in a cartridge 21 in which the adsorbent material, according to the invention, is covered with phospholipid membranes of a composition similar or identical to those of liposomes 10.

The adsorbent material preferably comprises activated-carbon granules 22 (or microspheres of synthetic plastic resin) fully covered with continuous phospholipid membranes 23. The liposomes 10 carrying the captured target molecules (toxins) are thus brought into close contact with filter 20 where, thanks also to the adsorbent capacity of granules/microspheres 22, the liposoluble substances captured by the liposomes are transferred into phospholipid membranes 23 and trapped inside filter 20, while liposomes 10, released from the retained molecules, are "regenerated" and used to form a "clean" stream 3 for recycling onto membrane 1.

Figures 2 and 3 show, schematically, two units (which have been used for "in vitro" experimental tests) according to the present invention. More specifically, the Figure 2 unit comprises a first hydraulic circuit 30 for the solution to be purified; a second hydraulic circuit 31 for the wash solution; and a filter 32 in turn comprising a casing 33, and a semipermeable membrane (not shown) of the same type as membrane 1, of a predetermined porosity, housed inside casing 33, and through which circuit 30 communicates hydraulically with circuit 31 to enable predetermined target molecules (e.g. toxins) in the solution for purification to diffuse into the wash solution. In the example shown, which, as stated, is an experimental "in vitro" unit, circuits 30 and 31 are closed circuits, each presenting a respective pump 34; circuit 30 presents a tank 35 for the solution to be purified, e.g. human or animal plasma or blood; and circuit 31 presents means for feeding liposomes into the flow of wash solution flowing in circuit 31, and comprising, in the example shown, a tank 36 preferably already containing a suspension of liposomes in an aqueous solution at the required concentration. In this case, therefore, the means for feeding the liposomes into circuit 31 provide for feeding them simultaneously with the wash solution, which therefore contains liposomes dispersed in suspension right from the start. Alternatively, tank 36 may contain only the liposomes (or a very high concentration of them in suspension), and may be provided with known means not shown (e.g. electronically controlled metering valves and/or pumps) for controlled release of the liposomes into circuit 31.

More specifically, filter 32 comprises a known pack of highly permeable, hollow cellulose fibers internally connected hydraulically to circuit 30 and housed inside casing 33, which in turn is hydraulically connected to circuit 31, so that the wash solution circulates inside casing 33, outside the hollow fibers, while the solution to be purified circulates inside the hollow fibers to reproduce the same pattern as in Figure 1, in which the semipermeable membrane comprises the lateral walls of the hollow fibers.

To operate according to the Figure 4 variation, circuit 31 may be fitted in series with a perfusion cartridge 38 housing filter 20 of adsorbent material; or filter 20 may be inserted directly inside casing 33 (to form a suitable cartridge) and adjacent to the hollow fibers forming semipermeable membrane 1. In either case, whereas circuit 31 must invariably be a closed circuit, circuit 30 may obviously be an "open" circuit closed by the patient's body, to enable the Figure 2 unit with the above variation to operate not only "in vitro", but also "in vivo", in which case the solution to be purified is drawn directly from the patient's bloodstream and fed back into it when purified, i.e. on issuing from filter 32.

The Figure 3 unit, any details of which similar or identical to those described are indicated for the sake of simplicity using the same numbering system, differs from the Figure 2 unit by providing for diafiltration as opposed to straightforward dialysis. That is, whereas circuits 30, 31 of the Figure 2 unit are so sized as to circulate the same volume of the two solutions (the one being purified and the wash solution), e.g. 100, 200 ml/minute, so that there is no pressure difference between the two sides of membrane 1, circuits 30a and 31a of the Figure 3 unit are so sized as to circulate different volumes of the two solutions, e.g. 300 ml of the solution to be purified in 30a, and 200 ml of the liposome wash solution in 31a.

Consequently, at the membrane of filter 32, a pressure gradient (TMP) is formed which provides not only for diffusion but also for convection through the membrane, and the solution in circuit 30a undergoes a weight loss due to transfer of the solutes (and part of the solvent) into the wash solution. Unlike circuit 31, therefore, circuit 31a is also provided in series with a second filter 40 featuring a semipermeable membrane like membrane 1, with pores smaller than the liposomes in the wash solution, and from which extends a branch 41 connected to circuit 30a immediately downstream from filter 32. As such, the part of the solution for purification flowing in circuit 31a due to the pressure gradient on filter 32 is recovered, purified and free of liposomes, in filter 40 and fed back into circuit 30a along branch 41 as a reinfusion solution to fully compensate the weight loss. In this case also, circuit 31a may present a cartridge 38 for generating the liposomes, or filter 20 of adsorbent material may be housed inside the casing of filter 32 as described previously.

Figure 5 shows, schematically, the main parts of a dialysis unit 100 for purifying blood, particularly in patients affected with organ failure, and which comprises an open circuit, in turn comprising a hemofiltration element 101 and a hemodialysis element 103 hydraulically in series with each other (cascade connected) between an input conduit 104 and an output conduit 105 for respectively connecting the unit to a known circuit (not shown) for drawing blood from and feeding purified blood back into the patient's body.

The blood to be purified flows in along conduit 104, flows through cascade elements 101 and 103, and flows out, purified, along conduit 105; the hemodialysis products (ultrafiltrate) formed in element 101 flow out along an output conduit 107; and a conduit 108 provides for feeding a reinfusion solution into the conduit 106 connecting element 101 to hemodialysis element 103, to partly or fully compensate the weight loss produced in the solution (blood) ultrafiltrated in element 101. More specifically, the reinfusion solution is formed by regenerating the ultrafiltrate extracted along conduit 107, for which purpose unit 100 comprises a regenerating device 110 in series with and connecting conduits 107, 108, and which may comprise a bare hemoperfusion carbon cartridge, e.g. a commercial Detoxil 2™ cartridge produced by Sorin Biomedica S.p.A. of Saluggia, Italy (as described EP-A-0 451 429), or a unit B identical to the one in Figure 2 or 3, except that, as opposed to being closed, the corresponding circuit 30 or 30a is open, e.g. at tank 35 which is eliminated, and the opposite ends of the circuit are connected respectively to conduits 107 and 108.

According to a variation of unit 100, the block 120 of elements inside the dot-and-dash rectangle may be replaced by the block 121 in the dot-and-dash rectangle in Figure 3, so that element 101 is replaced by filter 32, the input and output of which are connected respectively to conduits 107 and 108.

Hemodialysis element 103 is connected to a wash solution circuit 120 indicated in block form by the dotted line. According to a further variation, the conventional circuit 120 may be replaced by liposome circuit 31 in Figure 2, or by circuit 31a in Figure 3, complete with branch 41. In which case, elements 101 and 111 will obviously be used upstream from element 103.

Unit 100 therefore always comprises at least two hydraulic circuits separated by a dialysis or hemodialysis filter (101, 32 or 103); a first of the circuits contains a solution for purification drawn from the patient's bloodstream (directly or indirectly via another circuit with a convection filter such as 101); and a second of the circuits contains a wash solution with liposomes 10 in suspension. Like circuit 31a, the second circuit may also be provided in series with means 40, 41 for convection separating from the wash solution a mass of ultrafiltrate containing no liposomes or toxins, and for feeding it into the first circuit downstream from the filter.

In turn, the first circuit may comprise, upstream from the dialysis (or diafiltration) filter, a hemofilter such as 101 for convection separating a mass of ultrafiltrate from the patient's bloodstream, and may be branch connected, downstream from the hemofilter, to means for supplying a reinfusion solution. Which means may comprise a unit connected to the hemofilter, for receiving the ultrafiltrate and feeding it in series through an activated-carbon hemoperfusion cartridge such as 111, and then feeding it back into the first circuit as a reinfusion solution. Alternatively, hemoperfusion cartridge 111 may be series connected to the first circuit.

A number of practical embodiments of the present invention will now be described by way of example.

### EXAMPLE 1

- Liposome preparation:
   The liposomes are prepared from the following material:
   - 100% of over 99% pure L-α-phosphatidylcholine (PC) obtained from chicken egg whites and soya. The phospholipids used are obtained by GMP, are certified for pharmaceutical use, are free from lysophospholipids, hydroperoxides, hydroxides and fatty acids, as per spectrophotometric analysis, and are distributed by Avanti Polar Lipids (Alabama, USA).
   - synthetic ubiquinone/ubiquinol antioxidant made and distributed by Avanti Polar Lipids (Alabama, USA).

   The phospholipids and antioxidant, of which 0.5% of the total weight is incorporated in the phospholipids, are dissolved in ethanol in a flask, which is left under a hood to allow the solvent to evaporate; before the solvent evaporates completely, the flask is subjected to a stream of gaseous nitrogen to assist evaporation of the solvent and prevent the phospholipids and antioxidant from oxidizing; following evaporation, the phospholipids and antioxidant form a thin film at the bottom of the flask, which is inspected for blistering or other solvent residue; if detected, methanol or ethanol or chloroform is added to redissolve the film, and the evaporation process is repeated until a perfect film is formed; the film is then added a few ml of modified phosphate buffered saline (PBS) containing sodium phosphate 0.008M, potassium phosphate 0.002M, sodium chloride 0.14M, and 7.4 pH potassium chloride 0,01M (purchased from Pierce, Rockford Illinois, USA) - a few ml are normally enough to hydrate the phospholipids and dissolve the film; once the film is dissolved, working in a constant flow of nitrogen, the flask is sealed to leave nitrogen inside it, is agitated vigorously for 10 minutes, is inspected, and is again agitated if it still presents traces of film or phospholipid "lumps"; PBS is then added to obtain a phospholipid concentration of 3 to 10 mg per ml of PBS; and the phospholipids are collected in multilamellar vesicles (MLV).
   The MLV solution is then fed through a 10 ml capacity liposome extruder (LIPEX Biomembranes Inc., Vancouver, British Columbia, Canada) equipped with two disposable 25 mm polycarbonate filters of 100 or 200 nm pore size. The solution is fed through the extruder 10 times to ensure the formation of homogeneous liposomes, which are unilamellar with the same diameter as the filter pores (100 or 200 nm).
- Coated carbon preparation:
   About 50 gr of XEN 546 (Supelco) are placed in a 250 ml flask; 10 g of lecithin in 100% chloroform are added to the flask, and the solution is agitated gently under a stream of gaseous nitrogen; when dry, the carbon is placed in a desiccator containing a solution of K₂SO₄ which causes the phospholipids to collect into bilamellar layers; the carbon is then added to 200 ml of PBS and pumped through a polysulfone HFT-02 filter (BELLCO); when the filter is charged, the excess PBS is removed.

### EXAMPLE II

The liposome in PBS or PBS only solution is incubated with cow blood as shown in Table 1:

**TABLE 1**

| | |
|---|---|
| A | 1 ml liposomes + 1 ml blood |
| B | 0.5 ml liposomes + 1 ml blood + 0.5 ml PBS |
| C | 1 ml blood + 1 ml PBS |
| D | 1 ml liposomes + 1 ml blood + 2 µl chloroform |
| E | 1 ml liposomes + 1 ml blood + 5 µl chloroform |
| F | 1 ml liposomes + 1 ml blood + 10 µl chloroform |
| G | 1 ml blood + 1 ml PBS + 2 µl methanol |
| H | 1 ml liposomes + 1 ml blood + 5 µl methanol |
| I | 1 ml liposomes + 1 ml blood + 10 µl methanol |
| J | 1 ml liposomes + 1 ml blood + 2 µl ethanol |
| K | 1 ml liposomes + 1 ml blood + 5 µl ethanol |
| L | 1 ml liposomes + 1 ml blood + 10 µl ethanol |
| M | 1 ml PBS + 1 ml blood + 2 µl chloroform |
| N | 1 ml PBS + 1 ml blood + 2 µl methanol |
| O | 1 ml PBS + 1 ml blood + 2 µl ethanol |

The plasma is separated by centrifugation in 10 ml polypropylene tubes at 2,500 rpm for 15 minutes at room temperature; spectra of the plasma fraction (200 to 800 nm) are taken using a BECKMAN 7400 diode-matrix spectrophotometer; and specimens are taken at 0, 5, 15, 30 minutes, and 1, 6, 24 hours. No hemolysis or increase in absorbance is observed in the 234 nm wavelength (indicating lipid peroxidation) in the specimens containing liposomes (A and B), whereas all the specimens containing chloroform present extensive hemolysis.

### EXAMPLE III

In-circuit dialysis tests are performed using a circuit as in Figure 2, and circulating the following in circuit 30 (blood side):
A. cow blood
B. cow plasma
C. PBS + 4 g/l of albumin

The cow blood is procured fresh from the abattoir in a 5-liter bag containing 500 ml of heparinized normal saline solution prepared by adding 45 g of NaCl with 10 ml of sodium heparin stock solution (5.000 U heparin per ml of bidistilled water) to a final volume of 5 liters of bidistilled water; the blood is filtered through a porous fabric (saturated beforehand with the heparinized normal saline solution); the plasma is prepared by transferring the filtered blood into a 50 ml centrifuge tube rinsed beforehand with heparinized saline solution, and centrifuging it at 2,700 rpm for 15 minutes; the plasma is then transferred into another 50 ml centrifuge tube and either used fresh or stored at -20°C. Solution C is prepared by adding 4 g of V type albumin (SIGMA, Milan, Italy) per 100 ml of PBS, and is then agitated gently to prevent foaming or protein denaturation.
To the above solutions are added various concentrations of the following toxins:
PAF (20 ng/ml); bilirubin (10 ng/ml).
The toxins are added to the initial material, and the whole is divided into specimens to test the following wash solutions in circuit 31:
- control solution (PBS only);
- liposome solution;
- liposome solution with cartridge 38 equipped with a phospholipid-coated activated-carbon filter.

This ensures the same toxin concentration in all the tests.
The circuit 30 and 31 tests are conducted as follows:
The filter (32) used is a BELLCO HFT-02 high-flow polysulfone 0.44 m2 filter; the circuits are rinsed with 2 liters of normal saline solution, after which, the dialyzate side (circuit 31) is filled with 300 ml of dialyzate (PBS) or 300 ml of liposome solution (600 mg of liposomes in 300 ml of PBS), and the blood side (circuit 30) is filled with equal volumes of substances A (blood), B (plasma) and C (PBS + albumin solution). The PAF tests are conducted using solution C only, in that both plasma and blood contain an enzyme (acetylhydrolase) capable of degrading any added platelet activating factor. The bilirubin tests are conducted using blood, plasma and solution C, with similar results.
In the circuits, 75 ml/minute are circulated on the blood side (circuit 30) and 135 ml/minute on the dialyzate side (circuit 31); and the liposome tank is placed on an analytic balance at the end of the test to check no ultrafiltration has taken place.
Specimens from both circuits 30, 31 are taken at the predialysis stage after 1, 10 and 30 minutes, and 1, 3, 4, 6 and 24 hours.
The specimens are then analyzed to determine:
- Platelet activating factor:
   The PAF content is analyzed by preparing:
- a gelatin buffer (without Ca⁺⁺) of pH 6.5 (the pH is lowered using HCl 1N);
- a gelatin buffer (with Ca⁺⁺) of pH 7.4;
- EDTA 0.2 M of pH 7.2.
   A platelet suspension is prepared of 50 ml of rabbit blood (from a 2.5 kg rabbit) in a 50 ml test tube containing 1.3 ml of EDTA; and the platelet activating factor (PAF) is measured by adding 250 ml of gelatin buffer, 20 ml of platelet suspension and 10 to 30 ml of the test specimen. Controls include the liposome only solution (at concentrations from 0.1 to 1 mg/ml), the dialyzate alone (PBS), and standard PAF specimens (2-20 ng/ml).
   Platelet aggregation is monitored using an ELVI (Milan, Italy) detector; and the platelet activating factor is quantified according to the change in shape of the platelets, using the method described by TETTA et al. (C. Tetta, G. Segoloni, A. Pacitti, G. Regis, M. Salomone, E. Turello, G. Camussi, A. Vercellone, "The production of platelet activating factor during hemodialysis." Int. J. Artif. Organs 12: 766-772, 1989, Milan).
- Bilirubin measurement:
   Bilirubin is measured using a commercial SIGMA Chemicals 550-A kit according to the maker's instructions.

The test results are shown in Figures 6 and 7. As can be seen, using liposomes, either alone or in conjunction with a phospholipid-coated-carbon regenerating filter (cartridge 38), provides for a definite improvement in the removal of controlled toxins, in terms of both absolute quantity and rapidity.

## Claims

1. A dialysis unit for purifying blood, particularly in patients affected with organ failure, the unit comprising a first hydraulic circuit (30) for feeding a solution for purification derived from the patient's bloodstream; a second hydraulic circuit (31) for feeding an aqueous wash solution, and a filter (32) in turn comprising a semipermeable membrane (1) of predetermined porosity, and through which the first circuit (30) communicates hydraulically with the second circuit (31) to enable the toxins in the solution for purification to diffuse into the wash solution; characterized in that the second circuit (31) comprises a tank (36) containing liposomes (10) and means for feeding said liposomes (10) into said second circuit (31) to form a suspension of dispersed liposomes (10) in the aqueous wash solution.

2. A dialysis unit as claimed in Claim 1, characterized in that said means (36) for feeding liposomes into the second circuit (31) provide for supplying said liposomes (10) simultaneously with said wash solution, so as to supply the second circuit (31) with an already formed said suspension of dispersed liposomes.

3. A dialysis unit as claimed in Claim 1, characterized in that said means (36) for feeding liposomes into the second circuit (31) provide for controlled release of the liposomes (10) into the second circuit so as to form said suspension of dispersed liposomes in the second circuit (31).

4. A dialysis unit as claimed in Claim 2 or 3, characterized in that the second circuit (31) is provided, hydraulically in series, with a perfusion cartridge (38) comprising a filter (20) of adsorbent material covered with phospholipid membranes (23) compatible with those defining said liposomes (10); said wash solution and said liposomes dispersed in suspension in said wash solution flowing through said filter (20).

5. A dialysis unit as claimed in Claim 4, characterized in that said perfusion cartridge contains a filter (20) of activated-carbon particles covered with continuous phospholipid membranes (23) comprising at least one component in common with those of the phospholipid membranes of said liposomes (10).

6. A dialysis unit as claimed in any one of the foregoing Claims, characterized in that said second circuit (31) presents, in series, means (40) for separating by convection from said wash solution a mass of a first ultrafiltrate (41) devoid of said liposomes and said toxins, and for feeding said first ultrafiltrate (41) into the first circuit (30) downstream from said filter (32).

7. A dialysis unit as claimed in any one of the foregoing Claims, characterized in that said first circuit (30) comprises, in series, a hemoperfusion cartridge (110).

8. A dialysis unit (100) as claimed in any one of the foregoing Claims, characterized in that said first circuit comprises a hemofilter (101) for separating by convection from the patient's bloodstream a mass of a second ultrafiltrate (107), and is branch connected, downstream from the hemofilter (101), to means for feeding in a reinfusion solution (108).

9. A dialysis unit (100) as claimed in Claim 8, characterized in that said means for feeding in said reinfusion solution (108) are connected to the hemofilter (101), and provide for receiving said second ultrafiltrate and feeding it in series through a bare-activated-carbon hemoperfusion cartridge (110) and then back into the first circuit as a reinfusion solution.

## Patentansprüche

1. Dialysevorrichtung zur Reinigung von Blut, insbesondere bei Patienten mit einem Organversagen, wobei die Vorrichtung einen ersten hydraulischen Kreislauf (30) zum Zuführen einer Lösung zum Reinigen, die aus dem Blutstrom des Patienten stammt, einen zweiten hydraulischen Kreislauf (31) zum Zuführen einer wäßrigen Waschlösung, und einen Filter (32) umfaßt, der wiederum eine semipermeable Membran (1) von vorbestimmter Porosität umfaßt und durch den der erste Kreislauf (30) hydraulisch mit dem zweiten Kreislauf (31) in Verbindung steht, um es den Toxinen in der Lösung zum Reinigen zu ermöglichen, in die Waschlösung zu diffundieren, dadurch gekennzeichnet, daß der zweite Kreislauf (31) einen Behälter (36), der Liposome (10) enthält, und Mittel zum Zuführen der Liposome (10) in den zweiten Kreislauf (31) umfaßt, um eine Suspension aus dispergierten Liposomen (10) in der wäßrigen Waschlösung zu bilden.

2. Dialysevorrichtung nach Anspruch 1, dadurch gekennzeichnet, daß die Mittel (36) zum Zuführen von Liposomen in den zweiten Kreislauf (31) eine gleichzeitige Zuführung der Liposome (10) mit der Waschlösung zur Verfügung stellen, um so den zweiten Kreislauf (31) mit einer bereits gebildeten besagten Suspension an dispergierten Liposomen zu versorgen.

3. Dialysevorrichtung nach Anspruch 1, dadurch gekennzeichnet, daß die Mittel (36) zum Zuführen von Liposomen in den zweiten Kreislauf (31) eine kontrollierte Abgabe der Liposome (10) in den zweiten Kreislauf zur Verfügung stellen, um so die Suspension an dispergierten Liposomen in den zweiten Kreislauf (31) zu bilden.

4. Dialysevorrichtung nach Anspruch 2 oder 3, dadurch gekennzeichnet, daß der zweite Kreislauf (31) hydraulisch in Reihe mit einer Perfusionspatrone (38) zur Verfügung gestellt wird, welche einen Filter (20) aus Adsorptionsmaterial umfaßt, welches mit Phospholipidmembranen (23) bedeckt ist, die mit denen, welche die Liposome (10) definieren, kompatibel sind; wobei die Waschlösung und die in Suspension in der Waschlösung dispergierten Liposome durch den Filter (20) fließen.

5. Dialysevorrichtung nach Anspruch 4, dadurch gekennzeichnet, daß die Perfusionspatrone einen Filter (20) aus Aktivkohleteilchen enthält, die mit kontinuierlichen Phospholipidmembranen (23) bedeckt sind, welche mindestens eine Komponente wie die der Phospholipidmembranen der Liposomen (10) umfassen.

6. Dialysevorrichtung nach einem der vorhergehenden Ansprüche, dadurch gekennzeichnet, daß der zweite Kreislauf (31) in Reihe geschaltete Mittel (40) aufweist, zur Abtrennung durch Konvektion einer Masse eines ersten Ultrafiltrats (41) ohne den Liposomen und den Toxinen von der Waschlösung, und zum Zuführen des ersten Ultrafiltrats (41) in den ersten Kreislauf (30) stromabwärts des Filters (32).

7. Dialysevorrichtung nach einem der vorhergehenden Ansprüche, dadurch gekennzeichnet, daß der erste Kreislauf (30) eine in Reihe geschaltete Hämoperfusionspatrone (110) umfaßt.

8. Dialysevorrichtung (100) nach einem der vorhergehenden Ansprüche, dadurch gekennzeichnet, daß der erste Kreislauf einen Hämofilter (101) zur Abtrennung durch Konvektion einer Masse eines zweiten Ultrafiltrats (107) von dem Blutstrom des Patienten umfaßt, und über eine Zweigleitung stromabwärts des Hämofilters (101) mit Mitteln zum Zuführen einer Reinfusionslösung (108) verbunden ist.

9. Dialysevorrichtung (100) nach Anspruch 8, dadurch gekennzeichnet, daß die Mittel zum Zuführen der Reinfusionslösung (108) mit dem Hämofilter (101) verbunden sind und eine Aufnahme des zweiten Ultrafiltrats und dessen Zuführen in Reihe durch eine reine Aktivkohle-Hämoperfusionspatrone (110) und dann, als eine Reinfusionslösung, zurück in den ersten Kreislauf vorsehen.

## Revendications

1. Appareil de dialyse destiné à épurer le sang, en particulier chez des patients souffrant d'une insuffisance organique, l'appareil comprenant un premier circuit hydraulique (30) transportant une solution à épurer dérivée du courant sanguin d'un patient, un second circuit hydraulique (31) transportant une solution aqueuse de lavage, et un filtre (32) comprenant lui-même une membrane semi-perméable (1) de porosité prédéterminée et à travers lequel le premier circuit (30) communique hydrauliquement avec le second circuit (31) pour permettre aux toxines présentes dans la solution à épurer de se diffuser dans la solution de lavage ; caractérisé en ce que le second circuit (31) comprend un réservoir (36) contenant des liposomes (10) et un moyen pour introduire lesdits liposomes (10) dans ledit second circuit (31) de manière à former une suspension de liposomes (10) dispersés dans la solution aqueuse de lavage.

2. Appareil de dialyse tel que revendiqué dans la revendication 1, caractérisé en ce que ledit moyen (36) pour introduire les liposomes dans le second circuit (31) permet de délivrer lesdits liposomes (10) simultanément à ladite solution de lavage, de manière à délivrer au second circuit (31) une suspension déjà formée de liposomes dispersés.

3. Appareil de dialyse tel que revendiqué dans la revendication 1, caractérisé en ce que ledit moyen (36) pour introduire les liposomes dans le second circuit (31) permet une libération réglée des liposomes (10) dans le second circuit, de manière à former ladite suspension de liposomes dispersés dans le second circuit (31).

4. Appareil de dialyse tel que revendiqué dans la revendication 2 ou 3, caractérisé en ce que le second circuit (31) comporte, montée en série dans le parcours hydraulique, une cartouche de perfusion (38) comprenant un filtre (20) de matière adsorbante recouverte de membranes phospholipidiques (23) compatibles avec celles qui définissent lesdits liposomes (10) ; ladite solution de lavage et lesdits liposomes dispersés en suspension dans ladite solution de lavage s'écoulant à travers ledit filtre (20).

5. Appareil de dialyse tel que revendiqué dans la revendication 4, caractérisé en ce que ladite cartouche de perfusion contient un filtre (20) de particules de charbon activé recouvertes de membranes phospholipidiques continues (23) ayant au moins un composant en commun avec ceux des membranes phospholipidiques desdits liposomes (10).

6. Appareil de dialyse tel que revendiqué dans l'une quelconque des revendications précédentes, caractérisé en ce que ledit second circuit (31) comporte, en série, un moyen (40) pour séparer par convection de ladite solution de lavage une masse d'un premier ultrafiltrat (41) dépourvu desdits liposomes et desdites toxines, et pour envoyer ledit premier ultrafiltrat (41) dans le premier circuit (30) en aval dudit filtre (32).

7. Appareil de dialyse tel que revendiqué dans l'une quelconque des revendications précédentes, caractérisé en ce que ledit premier circuit (30) comporte, en série, une cartouche d'hémoperfusion (110).

8. Appareil de dialyse (100) tel que revendiqué dans l'une quelconque des revendications précédentes, caractérisé en ce que ledit premier circuit comporte un hémofiltre (101) pour séparer par convection du courant sanguin du patient une masse d'un second ultrafiltrat (107), et connecté par une dérivation, en aval de l'hémofiltre (101), à un moyen pour introduire une solution de réinjection (108).

9. Appareil de dialyse (100) tel que revendiqué dans la revendication 8, caractérisé en ce que ledit moyen pour introduire ladite solution de réinjection (108) est connecté à l'hémofiltre (101) et permet de recevoir ledit second ultrafiltrat et, successivement, de le faire passer à travers une cartouche d'hémoperfusion au charbon activé nu (110), puis de le renvoyer dans le premier circuit sous forme d'une solution de réinjection.
